# EUROPEAN PATENT SPECIFICATION

(11) **EP 0 764 426 B1**
(45) Date of publication and mention of the grant of the patent: **23.11.2005**
(21) Application number: 96115145.3
(22) Date of filing: 20.09.1996
(51) Int. Cl.: A61B 17/04, A61B 17/06, A61B 17/28

(54) **Vascular suturing apparatus**
Vaskuläre Nähvorrichtung
Appareil de suture vasculaire

(30) Priority: 22.09.1995 US 532908
(43) Date of publication of application: 26.03.1997
(73) Proprietor: United States Surgical Corporation, Norwalk, Connecticut 06856 (US)
(72) Inventor: Sherts, Charles R., Southport, CT 06490 (US); Scirica, Paul A., Huntington, CT 06486 (US)
(74) Representative: Marsh, Roy David

(56) References cited:
- EP-A- 0 535 906
- EP-A- 0 541 987
- EP-A- 0 647 431
- DE-B- 1 251 905
- FR-A- 337 579
- GB-A- 586 661
- US-A- 5 344 061

## Description

### BACKGROUND

### 1. Technical Field

The technical field relates generally to surgical suturing instrumentation and, more particularly, to surgical suturing apparatus for suturing vascular tissue sections together.

### 2. Description of Related Art

During many surgical procedures it is often necessary to join or even rejoin portions of vascular tissues or vessels to form an anastomosis. Various methods of joining vascular tissues to create an anastomosis are used, such as, for example, suturing, stapling or clipping the ends of the vessels together. Additionally, various stents may be used to join the vessels together and create the anastomosis. Where vessels are joined open end to open end it is termed an "end to end" anastomosis. In certain surgical procedures it is often desirable to join a free open end of one vessel to an incision in the side of another vessel to create an "end to side" anastomosis or even an incision in the side of another vessel to form "side-to-side" anastomosis.

In some instances suturing of vessels is preferred over stapling or clipping the vessels. Due to the small size of the vessels, a very small suturing needle is used having a length of suture material attached thereto to suture the vessels together. The suturing needle is typically grasped by a needle holder and passed through one vessel and then the opposite vessel. The procedure is repeated to thread or impart a series of stitches to the vessels to suture them together.

Because of the extremely small size of the suturing needle used, typically on the order of 0.25 mm (ten thousands of an inch) in diameter, handling problems may arise while manipulating the suturing needle through the vascular tissues. For example, upon piercing a vessel, the needle must be pushed through the vessel, released by the needle holder at one end of the needle and subsequently grasped at the opposite end of the needle to draw the needle and suture through the vessel thus requiring the release of the needle and suture during the procedure. Release of the needle is often undesirable and may pose problems in regaining control of the needle. To avoid this, it may become necessary to use two needle holders, one positioned on either side of the vessel, to continually grasp the needle, thereby requiring two hands to perform the operation. Additionally, precise control of the needle is often difficult when using typical needle holders. The small size of the needle also makes it difficult and time consuming to recover if dropped during the surgical procedure.

As noted above, the vascular sections to be sutured are typically extremely small. Suturing of such vascular tissue sections is often performed under magnification by equipping the surgeon with special magnifying glasses. The use of these magnifying glasses, while enlarging the view of the vascular tissues and needle, reduces the field of view within which the surgeon has to operate. Thus, as the needle and suture material are passed through the vessels and pulled to draw the suture material through, it often becomes necessary to move the needle holder and needle from the field of view. This may require the surgeon to look away from the field of view containing the vessel sections to be sutured and may present problems in repositioning the needle within the magnified field of view to form another stitch in the vascular tissues and increases the time required to suture the vessels together. Even when magnifying glasses are hot being used, the surgeon needs to look at the needle as it is moved away from the surgical site to pull the length of suture through the vessel and is passed to his other hand to be re-gripped. Due to the minute size of the vessels, it takes time for the surgeon to refocus on the surgical site to once again pass the needle and suture through the vessel. This repeated change of focus is time consuming and can place a strain on the surgeon's eyes.

An apparatus for suturing vascular tissue sections is disclosed in EP 0 705 568. Generally, the apparatus includes first and second arms pivotally connected together. Each arm has a needle receiving recess and a needle engaging member. The apparatus also includes a reciprocating mechanism connected to the needle engaging members and movable for alternately moving the needle engaging members into and out of the needle receiving recesses. The reciprocating mechanism is movable between a first position for advancing the first needle engaging member with respect to the first arm and a second position for advancing the second needle engaging member with respect to the second arm for alternately securing a surgical needle within the arms. A cam actuating lever is provided on one of the arms to automatically cam the reciprocating mechanism between the first and second positions upon closure of the first arm against the second arm.

EP 0 647 431 discloses a surgical apparatus which comprises two handles pivotally connected to an axially extending shaft at the distal end of which two jaw elements are connected for securing a surgical incision member. Two securing members are disposed within the longitudinal shaft and extend distally to both jaw elements for securing the incision member in either one of the jaws. An actuator is provided for reciprocal movement of the securing members at the distal end of the two handles. Upon squeezing of the handles and closing of jaws, the actuator has to be manipulated in order to secure the incision member to the other jaw element not previously engaged with the incision member.

FR 337 579 discloses an apparatus for holding a surgical needle which comprises a handle portion and distal from that two arms with corresponding recess holes for holding the surgical needle. Two tooth wheels are provided at the proximal end of the two arms which cooperate with a rod assembly being attached to the proximal ends of the two handles of the handle portion. Here, upon compressing of the two handles in a tweezer-like manner the rod assembly has to be manually brought in contact with the two tooth wheels which then advance one securing member in one of the arms and retracts the other securing member in the other of the two arms. Movement of the two handles is independent from the movement of the two securing members for the surgical needle.

Thus, it would be advantageous to have a surgical suturing apparatus and a method of suturing vessels which are particularly suited to suturing vascular tissues in anastomosis procedures. It would further be advantageous to have a surgical suturing apparatus which is operable with a single hand and is capable of maintaining precise and constant control of the needle as it is passed from one needle holding arm of the apparatus to another to avoid release of the needle during the suturing operation. It would also be advantageous to have a surgical suturing apparatus which is capable of suturing vascular tissue sections together with limited hand, apparatus and needle movement in order to maintain the entire suturing operation within a restricted field of view.

### SUMMARY

The invention relates to a surgical apparatus as defined in claim 1.

Preferred embodiments of the invention are defined in the dependent claims.

The disclosed surgical apparatus for suturing vascular tissue sections and includes a first arm having a first needle receiving recess and a first needle engaging member or blade mounted for movement with respect to the first arm. A second arm is provided and is mounted for movement with respect to the first arm and has a second needle receiving recess, the second arm having a second needle engaging member or blade mounted for movement with respect to the second arm. There is also provided a reciprocating mechanism connected to the first and second needle engaging members and movable for alternately moving the first and second needle engaging members into and out of the first and second needle receiving recesses to secure a surgical needle therein. The reciprocating mechanism is movable between a first position advancing the first needle engaging member into engagement with the surgical needle and a second position advancing the second needle engaging member into engagement with the surgical needle. A camming member or lever can be operatively associated with one of the arms for automatically camming the reciprocating mechanism between the first and second positions upon full closure of the first arm against the second arm.

Preferably, the reciprocating mechanism includes a toggle wheel rotatably affixed the arms and having first and second camming surfaces such that an initial closure of the arms forces the camming member into engagement with the first camming surface to move the toggle wheel to the first position. A subsequent closure of the arms forces the camming member into engagement with the second camming surface to move the toggle wheel to the second position.

Another example of the surgical apparatus for suturing vascular tissue sections comprises first and second arms and first and second jaws positioned therein. A needle engaging member is mounted for movement in each jaw to enable clamping or release of the needle within the jaw. A camming member is provided to automatically move a reciprocating mechanism to slide the needle engaging member between the clamping and release position. A ratchet mechanism is provided to prevent opening of the arms until the stroke is completed to transfer the needle between the jaws and a jaw retaining mechanism locks the jaws together during transfer of the needle. A quick release mechanism may also be provided to enable the needle to be readily disengaged from the instrument and reloaded or replaced.

The apparatus of the present invention is useful in a method of threading a suture through a vascular tissue section which includes the step of providing an apparatus having a first arm with a first needle receiving recess and a second arm with a second needle receiving recess, the first and second arms movable toward and away from each other. The method also includes the steps of holding a surgical needle within the second needle receiving recess, positioning the first and second arms about a first vascular tissue section to be sutured, moving the second arm toward the first arm such that the surgical needle pierces the first vascular tissue section and enters the first needle receiving recess and releasing the surgical needle from the second needle receiving recess and holding the surgical needle within the first needle receiving recess. The method also includes the step of moving the first arm away from the second arm to draw a suture affixed to the surgical needle at least partially through the first vascular tissue section.

### BRIEF DESCRIPTION OF THE DRAWINGS

Various embodiments are described herein with reference to the drawings, wherein:
FIG. 1 is a perspective view of an embodiment of a vascular suturing apparatus showing the jaws in the closed position;
FIG. 2 is an exploded perspective view of the vascular suturing apparatus of FIG. 1;
FIG 3 is a cross sectional view of the apparatus taken along lines 3-3 of FIG. 1;
FIGS. 4 and 5 are perspective views of each side of the toggle wheels of the apparatus;
FIG. 6 is a cross sectional view of a portion of the apparatus showing the jaws in the open position, the surgical needle secured in the lower jaw, and the toggle mechanism in the initial position;
FIG. 7 is a cross-sectional view taken along lines 7-7 of FIG. 6;
FIG. 8 is an enlarged cross-sectional view taken along lines 8-8 of FIG 6 showing securement of the needle in the lower jaw;
FIG. 9 is a side view in partial cross section of the apparatus with the jaws in the open position showing the side opposite that shown in FIG 1 to illustrate the initial position of the jaw retaining mechanism;
FIG. 10 is a side view similar to FIG. 9 showing the initial engagement of the jaw retaining mechanism;
FIG 11 is a cross sectional view similar to Fig. 6 with the jaws in the closed position but prior to actuation of the toggle mechanism and engagement of the ratchet mechanism;
FIG. 12 is a cross sectional view similar to FIG. 11 illustrating engagement of the ratchet mechanism but prior to reciprocation of the toggle mechanism;
FIG. 13 is a broken longitudinal cross sectional view of parts of the apparatus illustrating initial movement of the toggle mechanism to initiate release of the needle from the lower jaw and further showing engagement of the ratchet mechanism;
FIG. 13A is an enlarged side view showing the engagement of the jaw retaining mechanism;
FIG. 13B is an enlarged cross sectional view taken along lines 13B-13B of FIG 13 showing securement of the needle in the lower jaw;
FIG. 14 is a view similar to FIG. 13 illustrating reciprocation of the toggle mechanism to a second (counterclockwise) position to release the needle from the lower jaw and secure it in the upper jaw, and further showing the release of the ratchet mechanism;
FIG. 14A is a view similar to FIG 13A showing movement of the jaw retaining mechanism from an engaged to a release position;
FIG. 14B is a cross sectional view taken along lines 14B-14B of Fig. 14 illustrating release of the needle from the lower jaw;
FIG. 15 is a sectional view similar to FIG. 6 illustrating the jaws in the open position and the needle retained in the upper jaw; and
FIG. 16 is an enlarged cross sectional view of the needle release mechanism.
FIGS. 17A and 17B are enlarged views of an alternate suturing apparatus distal end and associated needle engaging member configuration;
FIGS. 18A and 18B are enlarged views of another alternate distal end and needle engaging member configuration;
FIGS. 19A and 19B are enlarged views of a further alternate distal end and needle engaging member configuration for use with a suturing needle having a hole at least partially therethrough;
FIG. 19C is a side plan view of a double pointed suturing needle having a hole at least partially therethrough;
FIGS. 20A and 20B are enlarged views of yet another alternate distal end and needle engaging member arrangement;
FIG. 21 is an enlarged view of an alternate suturing apparatus distal end slotted for ease in manipulation of a length of suture affixed to a surgical needle;
FIGS. 22A and 22B are enlarged views of another alternate distal end and needle engaging member configuration designed for easy loading of a surgical needle;
FIGS. 23A and 23B are enlarged views of an alternate easy load style distal end and needle engaging member configuration;
FIGS. 24A and 24B are enlarged views of still another alternate easy load style distal end and needle engaging member configuration;

### DESCRIPTION OF PREFERRED EMBODIMENTS

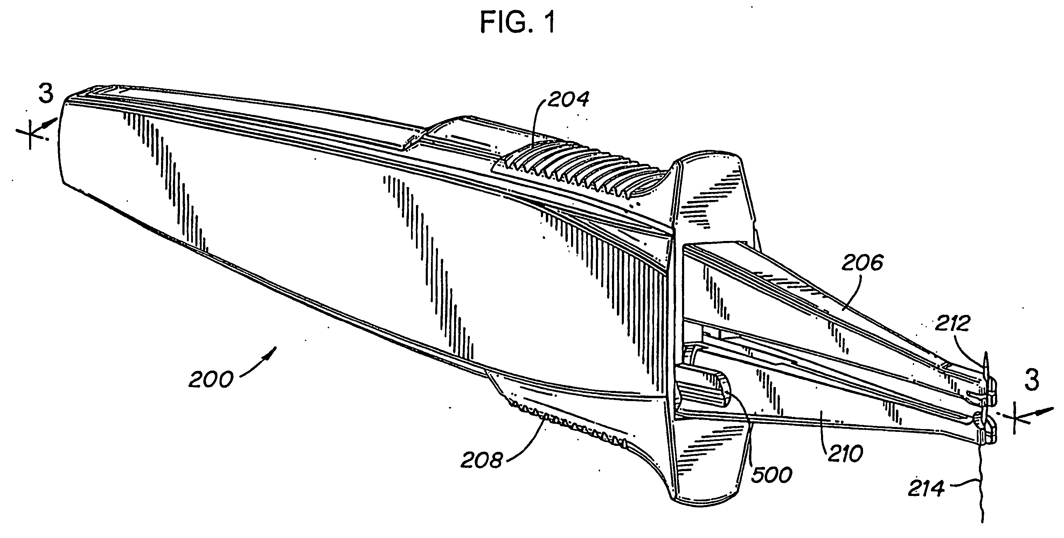

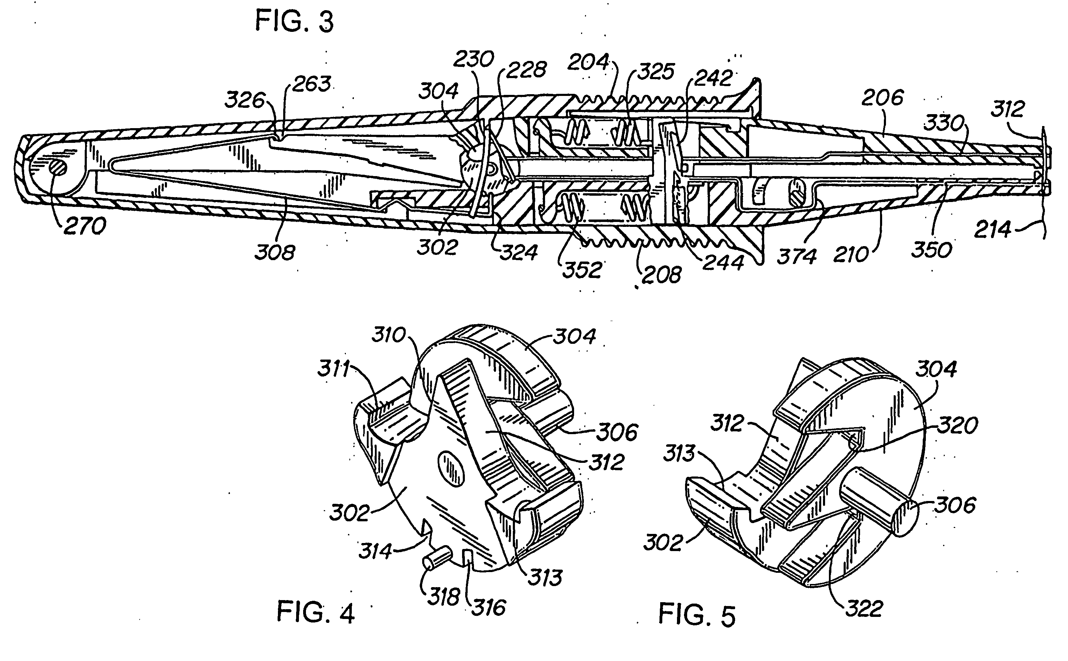

A vascular suturing apparatus is illustrated in Figs. 1 to 16. The apparatus of this embodiment provides automatic transfer of the surgical needle between the two jaws, accomplished with a single hand of the user.

With initial reference to Fig. 1 which shows the apparatus in the closed position in order to transfer the surgical needle 212, apparatus 200 has an upper arm or housing 204 supporting an upper jaw 206 and a lower arm or housing 208 supporting a lower jaw 210. As shown, arms 204, 208 preferably have a knurled gripping surface and are configured to be grasped by the surgeon in a tweezer-like manner to facilitate control of the apparatus for suturing the tissue. Lower arm 208 and lower jaw 210 remain fixed as upper arm 204 and upper jaw 206 move relative thereto to pass the surgical needle 212, with attached suture 214, between jaws 206, 210.

As can be appreciated, the terms "upper" and "lower" as used herein refer to the orientation of the instrument shown in Figure 1; clearly if the orientation of the instrument changes, these designations will change.

Surgical needle 212 preferably has a length of approximately 2.5 mm to 12.7 mm (.1 to .5 inches) and a diameter of approximately 0.25 mm to 0.64 mm (ten thousandths to .025 of an inch) which are particularly suited for vascular surgery. The needle 212 can be straight as shown, although other needle sizes and configurations, such as curved needles, are also contemplated. Preferably the surgical needle and suture have substantially equal diameter.

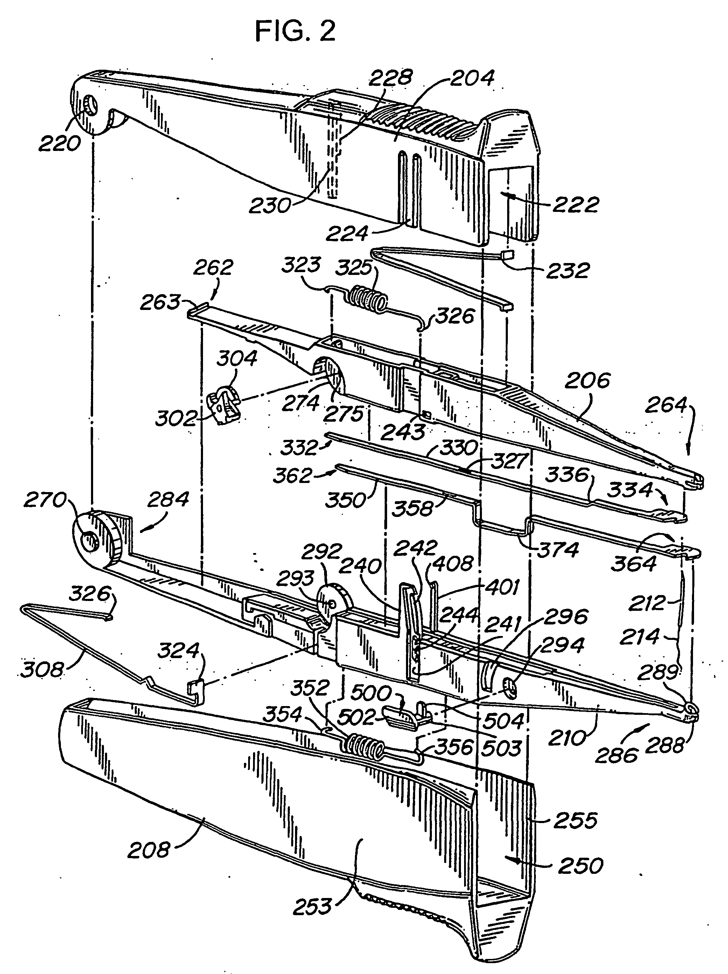

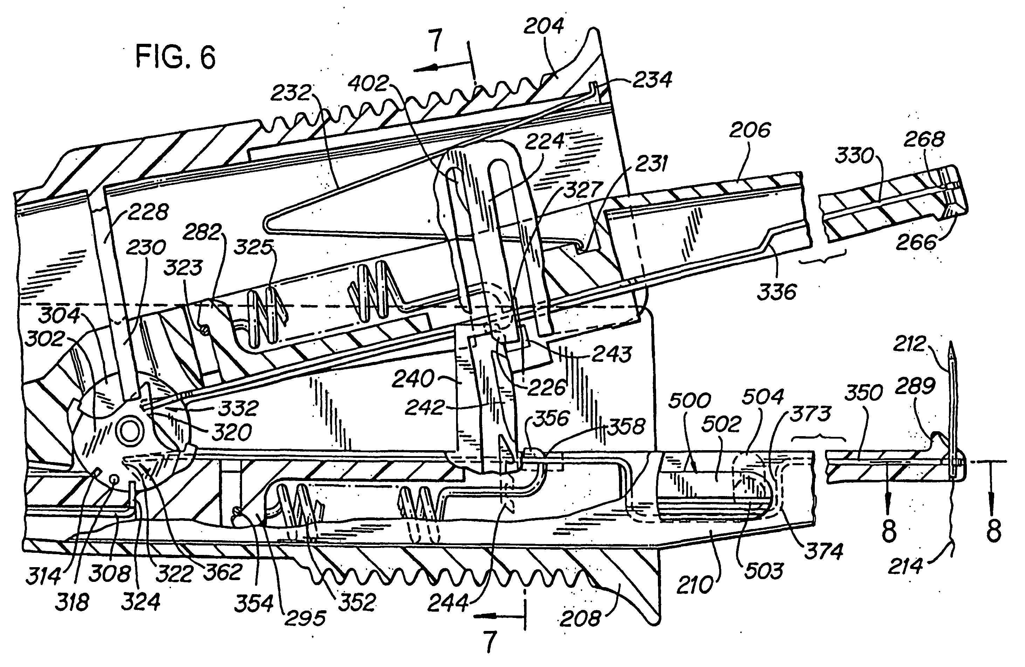

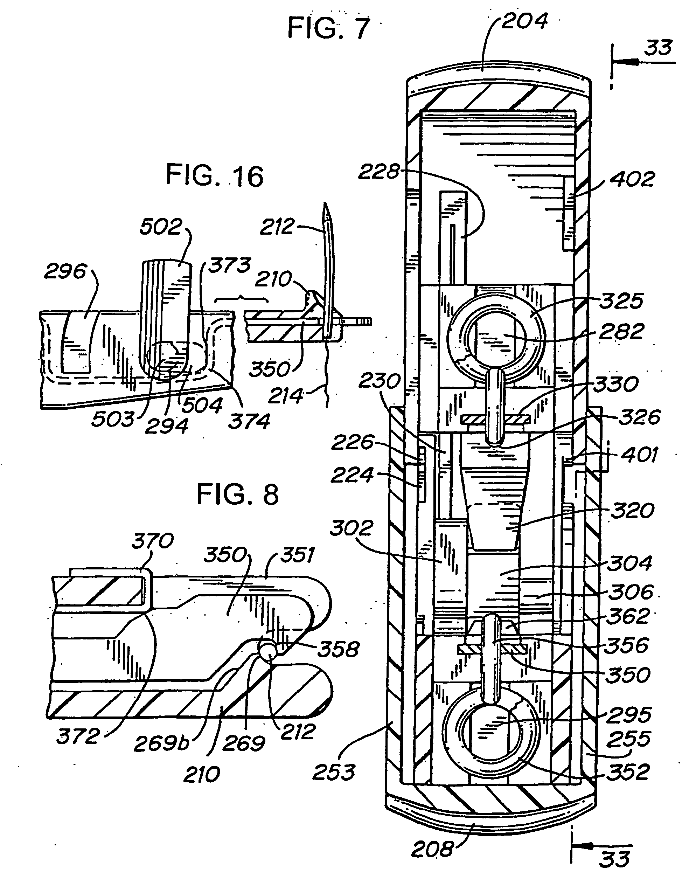

Referring to Figures 2 and 6, upper arm 204 is dimensioned for partial insertion within a cavity 250 formed in lower arm 208 (see also Fig. 7). Cavity 222 of upper arm 204 is dimensioned and configured to receive upper jaw 206. Aperture 220 at the proximal end receives pivot pin 270 of lower jaw 210 to enable pivotal movement of jaw 206 with respect to jaw 10. Upper arm 204 is biased away from upper jaw 206, i.e. biased to the open position, by spring 232 which has a first end seated within slot 234 of upper arm 204 and a second end abutting surface 231 of upper jaw 206. Spring 232 can alternatively be in the form of a vertically oriented coil spring or other configuration for biasing the arm 204 away from upper jaw 206. Upper arm 204 includes a toggle finger 228 with a flexible release finger 230 for actuation of the wheels 302, 304 of the toggle mechanism to effect transfer of the needle between the jaws 206, 210. The toggle mechanism reciprocates the needle securing blades 330, 350 to alternately secure and release the surgical needle 212 from the respective jaw. Upper arm 204 also includes a ratchet finger 224 having a pawl 226 on its inner surface (not shown in Figure 2) which forms part of a ratchet mechanism for retaining the upper arm 204 and lower jaw 210 together during transfer of the surgical needle between the jaws 206, 210. Thus, jaws 206, 210 cannot be opened until the stroke is completed and the needle is safely secured in one of the jaws, thereby preventing premature opening of the jaws and loss of the needle before it is properly transferred to the opposing jaw. The ratchet and toggle mechanisms are discussed in detail below.

Lower arm 208 of apparatus 200 has a cavity 250 formed between upstanding walls 253, 255 to receive lower jaw 210 as well as a portion of upper arm 204 (see also Fig. 7) when upper arm 204 is squeezed to the closed position. Lower jaw 210 and lower arm 208 remain fixed with respect to each other and with respect to upper arm 204 and upper jaw 206. Both lower and upper arms 204, 208 preferably taper in height towards their proximal end. It is also contemplated that alternately, both arms and jaws could pivot with respect to each other.

Upper jaw 206, as shown in Fig. 2, has a distal end 264 designed to receive surgical needle 212 in recess 266 and cooperates with upper blade 330 to frictionally clamp the needle in a manner described below. Slot 268 is configured to receive the upper blade 330 which is also described below. A coil spring 325, which biases upper blade 330 proximally to a needle securing position, is seated within upper jaw 206 such that proximal hook 323 wraps around post 282 and distal hook 326 extends through slot 327 in upper blade 330, best shown in Figure 6.

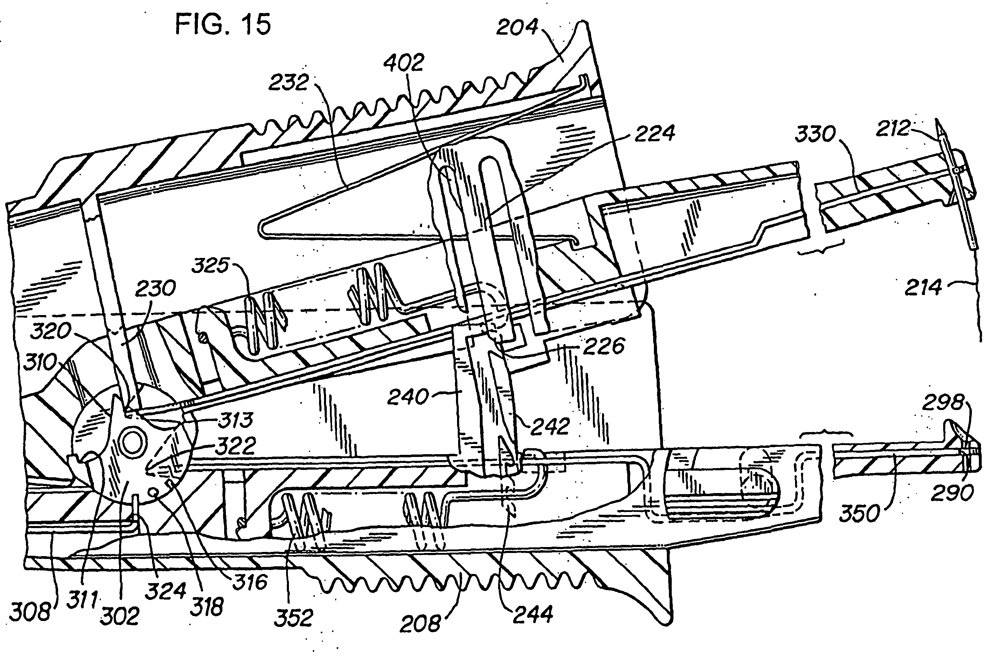

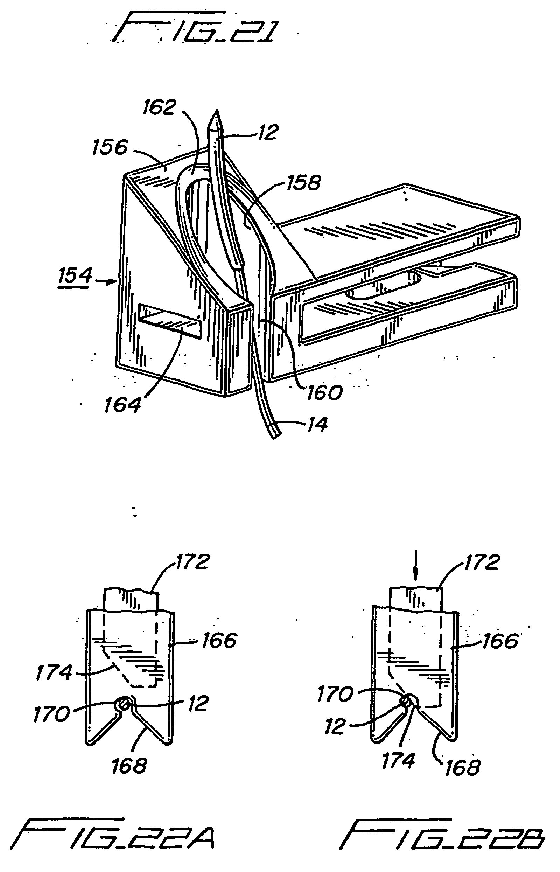

Lower jaw 210 has a pivot pin 270 at its proximal end 284 to engage aperture 220 of upper arm 204 to provide for pivotal movement of arm 204. Distal end 286 has a recess 288 for receiving the surgical needle 212 and a slot 290 for the lower blade 350 (Fig. 15). The lower blade 350 is biased proximally by coil spring 352 which, as shown in Fig. 6, has a proximal hook 354 wrapped around post 295 of lower jaw 210 and distal hook 356 extending through slot 358 in the lower blade 350. The distal end 286 is preferably configured with an inwardly sloped projecting portion 289. This is similar to the configuration of Fig. 21 which is described below.

Support 292 formed in lower jaw 210 has a hole 293 for receiving the mounting pin of the wheels 302, 304 of the toggle mechanism. Aperture 294 and slot 296 are configured to mount release button 500, the function of which is described below.

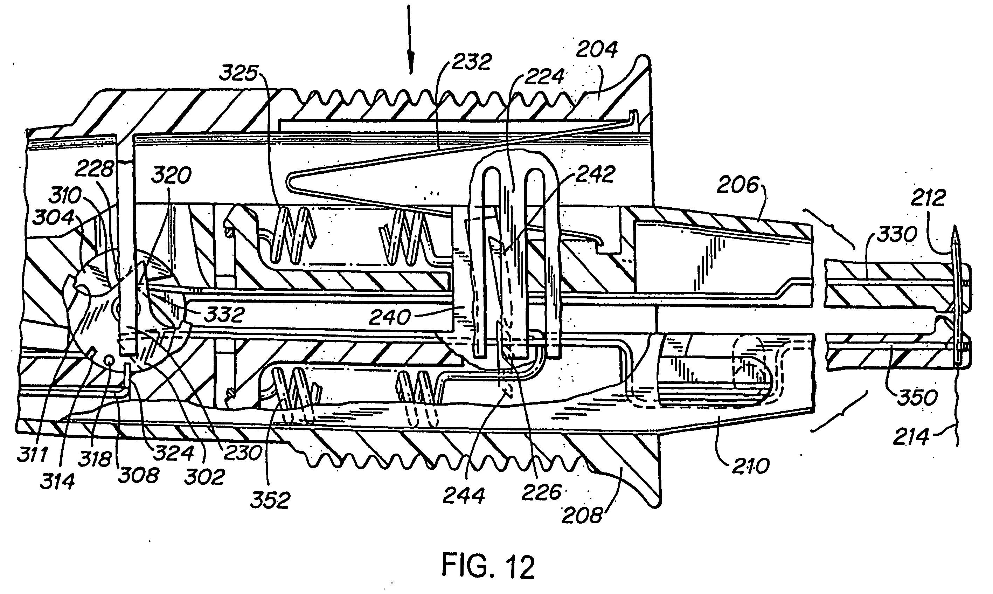

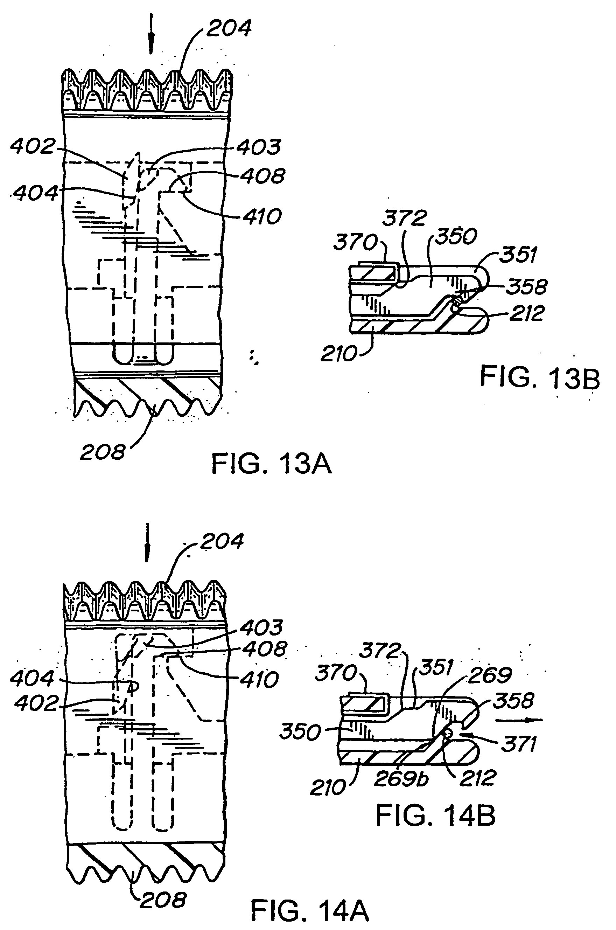

With continued reference to Figures 2 and 6, a support leg 240 extends upwardly from one side of lower jaw 210 and forms part of the ratchet mechanism to prevent separation of the upper arm 204 and lower jaw 210 until the needle 212 is securely held in one of the jaws. More particularly, a rack 244 is positioned within cutout portion 241 of lower jaw 210 and below projection 242 of support leg 240. As upper arm 204 is initially moved towards lower arm 208 to a closed position, ratchet finger 224, carrying pawl 226. on its inner surface, is initially biased distally by projection 242 such that pawl 226 extends into recess 243 of upper jaw 206. When the arms 204, 208 are further closed to a position where pawl 226 has traveled below projection 242, ratchet finger 224 snaps back in a proximal direction so that pawl 226 engages rack 244 as shown in Figure 12. Once engaged, the upper arm 204, and thus the upper jaw 210, cannot separate from the lower arm 206 and lower jaw 210 until the arms are fully closed and the pawl 226 completes its travel below the rack. (see Fig. 14) This completion of travel occurs only after the toggle mechanism has been fully actuated to release the needle 212 from one of the jaws and to secure the needle in the other jaw. Thus, separation of the jaws 206, 210 until the needle is safely transferred is prevented. The ratchet mechanism and its timing is discussed in more detail below in the description of the sequence of operation of the instrument.

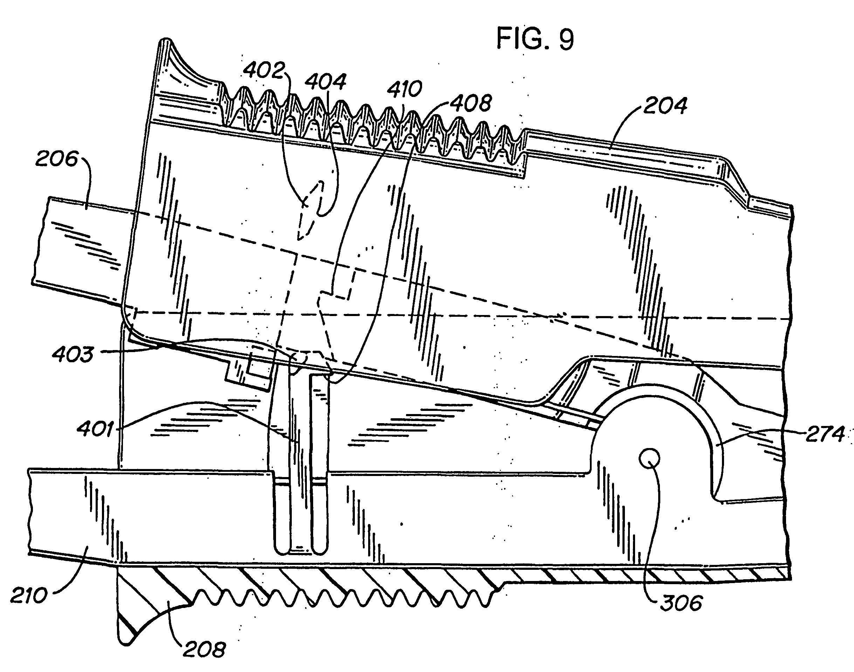

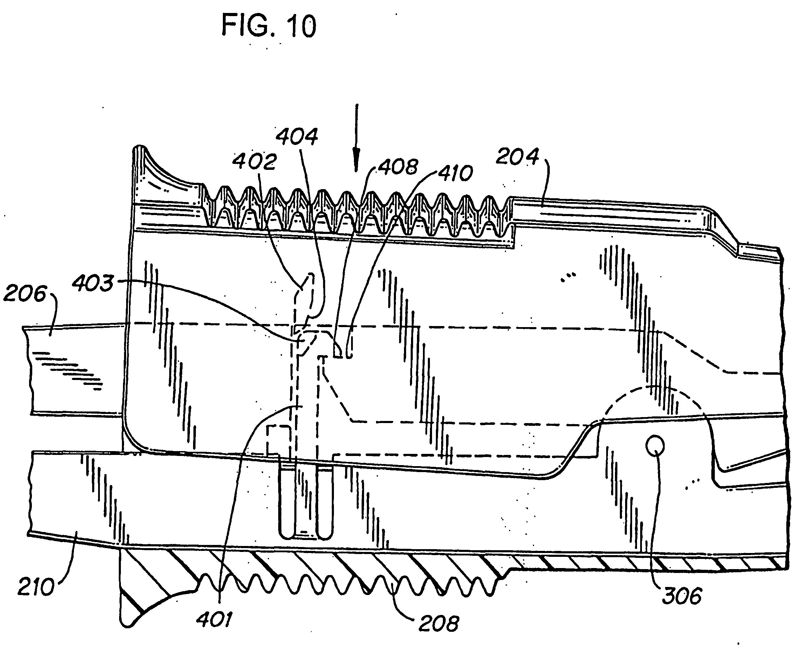

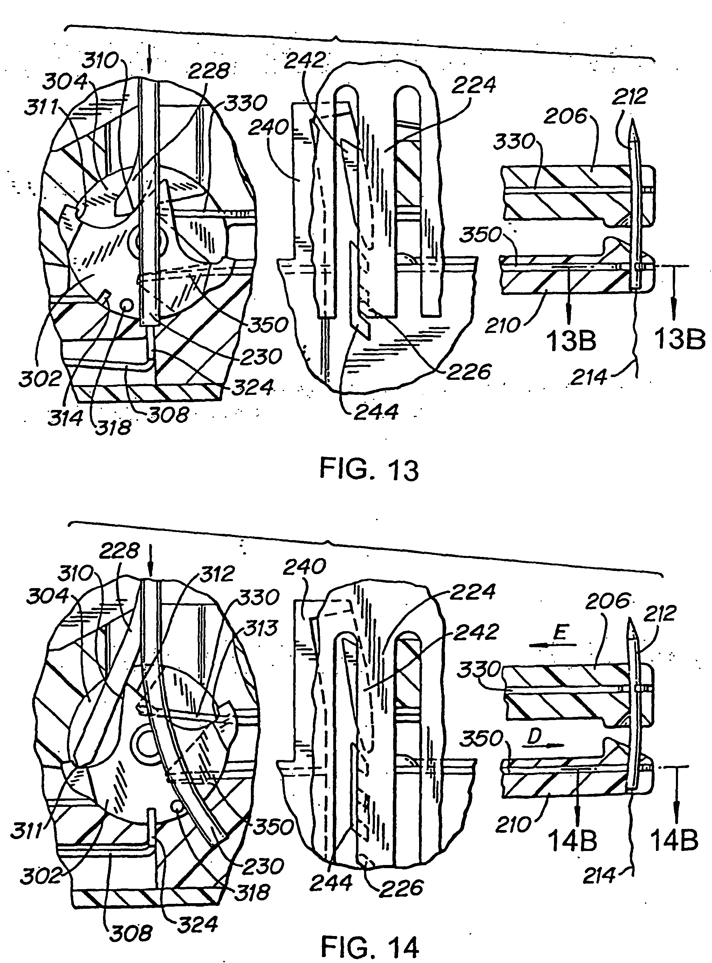

On the side of lower jaw 210 opposite the ratchet finger 224 is retaining arm 401 of the jaw retaining mechanism. Retaining arm 401 extends upwardly and substantially parallel to ratchet finger 224 and as shown in Fig. 9, has a ledge or hook 408 and a projection 403 on its outer surface. When the upper arm 204 is moved toward the lower arm 208, ledge 408 of retaining arm 401 slightly engages a shelf 410 formed in an outer wall of the upper jaw 206 (see Fig. 10). Upon further movement of upper arm 204 towards the lower arm 208, the camming surface 404 of camming projection 402, which is formed in an inner wall of upper arm 204, engages the projection 403 to force retaining arm 401 proximally. Thus, ledge 408 is moved further on shelf 410 and is held in this engaged position as shown in Fig. 13A. This effectively locks the upper and lower jaws 206, 210 together in a parallel position while upper arm 204 can complete its movement to the closed position to actuate the toggle mechanism to effect transfer of the surgical needle 212 to the opposing jaw. Figure 14A illustrates release of the retaining arm 401. As illustrated, when camming projection 402 slides past projection 403 after the arm 204 has been moved to the closed position, retaining arm 401 springs back to its normal distal position. This disengages retaining arm 401 from shelf 410 to allow the upper jaw 206 to return to the open position.

Turning now to the needle securing (clamping) blades, and referring first to the lower blade 350 and Figures 2, 6 and 8, lower blade 350 is slidably mounted within lower jaw 210 for movement between a proximal position for clamping the surgical needle 212 and a distal position for releasing the needle 212. More specifically, distal end 364 has a recess defined by locking edge 358 which presses needle 212 against surface 269 of the lower jaw 210. When in the proximal clamping position, coil spring 352 biases the lower blade 350 in a proximal direction such that biasing clip 370 (or alternately a biasing pin) engages angled surface 372 of the blade 350 to bias it into engagement with the needle 212 and to force it against surface 269 of lower jaw 210 to securely clamp the needle between the lower blade 350 and the lower jaw 210. When in the distal release position, the angled surface 372 is no longer in contact with biasing clip 370 and therefore the distal end 364 of blade 350 springs into slot 351 of lower jaw 210 and is also pushed into slot 351 by surface 269b. As can be seen in Fig. 14B, when positioned in the slot 351, a sufficient gap 371 is provided to facilitate release of the needle.

A bend 374 is formed in the lower blade 350 to cooperate with release button 500 in the manner described below.

Upper blade 330 functions to retain the surgical needle 212 in the upper jaw 206 in an identical manner as lower blade 350. That is, the distal end 334 of upper blade 330 has a recess defined by a locking edge to clamp the surgical needle against a raised surface (not shown) of upper jaw 206. Upper blade 330 is biased proximally by coil spring 325 to a needle clamping position. A biasing clip or pin (not shown) biases the blade into engagement with the needle in the same manner as biasing clip 370 of lower needle securing blade 350. Step 336 provides the blade 330 with additional flexibility.

The movement of the upper and lower blades 330, 350 are controlled by the toggle mechanism. The proximal end 362 of lower blade 350 tapers in width and in the illustrated embodiment angles downwardly to abut a portion of wheel 304 of the toggle mechanism. Similarly, the proximal end 332 of upper blade 330 tapers in width and angles downwardly to abut another portion of wheel 304 of toggle mechanism. (It should be appreciated that alternately the downward angle could be eliminated and the proximal end of blades 330, 350 can be straight.) Consequently, as described in detail below, rotation (pivoting) of abutment wheel 304 slides the upper and lower blades 330, 350 between proximal and distal positions to alternately clamp and release the surgical needle 212 from the respective jaw.

Turning now to the reciprocating or toggle mechanism, and with particular reference to Figs. 2-6, the toggle mechanism includes a toggle wheel 302 and a pivotable blade abutment wheel 304, both of which are rotatably mounted on mounting pin 306 within annular cavity 274 of upper jaw 206. Mounting pin 306 is fixedly secured in holes 275 and 293 of upper and lower jaws 206, 210, respectively. Toggle wheel 302 has a first angled camming surface and a first ledge designated by reference numerals 310, 311, respectively, and a second angled camming surface and second ledge designated by reference numerals 312, 313. Toggle wheel 302 further includes first and second locking notches 316, 314 and a knock-off pin 318 positioned therebetween. A locking spring 308 has a perpendicularly extending locking leg 324 engagable with one of the locking notches 314, 316 depending on the position of the toggle wheel 302. The opposite leg 326 of the locking spring 308 is seated within transverse slot 263 of upper jaw 206. This locking spring 308 prevents (blocks) movement of the toggle wheel 302 if the upper and lower arms 204, 208 are not in the closed position because locking spring 308 is disengaged (unlocked) from the respective locking notch only when upper arm 204 is moved to the closed position. By locking the toggle wheel 302, premature release of the needle 212, i.e. release of the needle before the opposing jaw has secured the needle, is prevented.

Blade abutment wheel 304 has a first V-notch 320 which abuts the proximal end of upper blade 330 and a second V-notch 322 which abuts the proximal end of lower blade 350. As blade abutment wheel 304 rotates clockwise as viewed in Fig 6, it slides upper blade 330 slightly distally, overcoming the force of coil spring 325, to release the surgical needle 212 from the upper jaw 206. Such clockwise movement simultaneously releases the distal force on coil spring 352 and allows coil spring 352 to bias lower jaw blade 350 proximally, thereby clamping the needle against the raised surface 269 of lower jaw 210 in the manner described above. Conversely, counterclockwise movement of abutment wheel 304 allows coil spring 325 to bias upper blade 330 proximally into the needle clamping position and overcomes the force of coil spring 352 to force lower blade 350 distally into a needle release position.

Movement of blade abutment wheel 304 is caused by the pivotal movement of toggle wheel 302. When the upper arm 204 is moved to the closed position, toggle finger 228 rides along the camming surface of toggle wheel 302 until it engages the ledge to rotate the wheel 302. As toggle finger rides along the camming surface, flexible release finger 230 engages locking leg 324 of locking spring 308 to force it out of engagement with the locking notch. More particularly, if the needle 212 is secured in the lower jaw 210 as shown in Fig. 6, sufficient depression of the upper arm 204 will cause toggle finger 228 to ride along first angled camming surface 310 as release finger 230 engages leg 324 of locking spring 308 to force it out of first locking notch 316. This is shown in Fig. 13.

As upper arm 204 is further moved (depressed) to the closed position, toggle finger 228 engages first ledge 311 causing toggle wheel 302 to rotate counterclockwise as shown in Fig. 14. This counterclockwise rotation of toggle wheel 302 causes abutment wheel 304 to rotate in the same direction, thereby sliding upper blade 330 proximally and lower blade 350 distally to clamp the needle 212 in the upper jaw 206 and release it from the lower jaw 210. Note that as toggle wheel 302 is rotated to its counterclockwise position, knockoff pin 318 keeps release finger 230 out of the way so that locking leg 324 of locking spring 308 can engage the second locking notch 314 to retain toggle wheel 302 (and abutment wheel 304) in the second (counterclockwise) position.

When the arms 204, 208 are opened and once again moved to the closed position in order to transfer the needle 212 back to the lower jaw 210, toggle leg 228 engages second camming surface 312 and second ledge 313 to rotate the toggle wheel 302 in a clockwise direction. Release finger 230 will force locking spring 308 out of engagement with the second locking notch 314 to enable it to re-engage the first locking notch 316. Clockwise rotation of toggle wheel 302 will cause clockwise rotation of abutment wheel 304 to slide upper blade 330 to a distal release position and allow lower blade 350 to return to its proximal clamping position.

The operation of the apparatus 200 will now be described. As shown in Fig. 6 the arms 204, 208 and jaws 206, 210 are in the open position with surgical needle 212 secured in the lower jaw 210 and spring 232 biasing the upper arm 204 away from the upper jaw 206. In this position, toggle wheel 302 and abutment wheel 304 are in the clockwise position such that lower blade 350 is biased proximally by coil spring 352 to clamp the surgical needle 212 in lower jaw 210 and upper blade 330 is forced into a distal (release) position by abutment wheel 304. Also note that locking leg 324 of locking spring 308 is engaged in the first locking notch 316 and release finger 230 is spaced therefrom. Furthermore, in this position, pawl 226 of the ratchet mechanism is spaced from projection 242 and rack 244 of leg 240, thus allowing free movement of the arms. Also, in this position, shown in Fig. 9, shelf 410 of upper jaw 206 is spaced from ledge 408 of retaining arm 401.

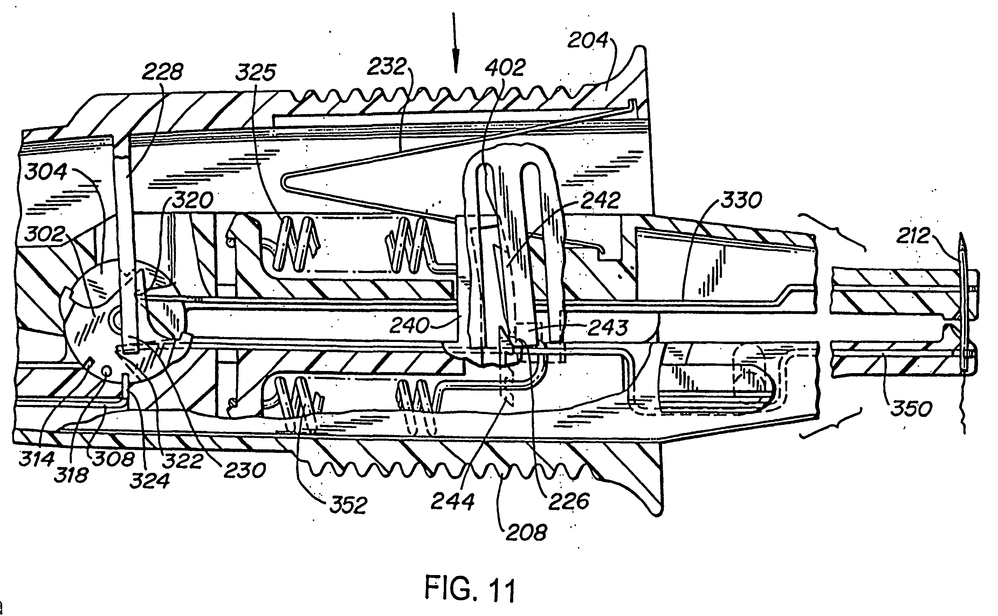

When it is desired to initiate suturing of the tissue and transfer of the surgical needle 212 from the lower jaw 210 to the upper jaw 206, arms 204, 208 are squeezed to move upper arm 204 and upper jaw 206 towards fixed lower arm 208 and lower jaw 210. Initially, upper arm 204 and jaw 206 move together towards lower jaw 210, with upper arm 204 remaining spaced from upper jaw 206 due to the biasing force of spring 232 and the engagement of pawl 226 of ratchet finger 224 in recess 243 of upper jaw 206. As the upper and lower jaws 206, 210 are moved to a substantially closed position, parallel to one another as shown in Fig. 11, release finger 230 has moved towards the locking spring 308 but remains out of engagement therewith and toggle finger 228 remains out of engagement with toggle wheel 302. Pawl 226 has almost completed its travel along projection 242 but remains out of engagement with rack 244 as it is biased into recess 243 of upper jaw 206 by projection 242. In this parallel position of the jaws, ledge 408 of retaining arm 401 has engaged shelf 410 of upper jaw 206 but is not securely held thereon because camming projection 402 remains out of engagement with projection 403 of retaining arm 401, as shown in Fig. 10.

Further depression of upper arm 204 in the direction of the arrow, moves it towards upper jaw 206 as upper jaw 206 has no more room to move and upper arm 204 begins to overcome the biasing force of spring 232. In this position illustrated in Fig. 12, release finger 230 still remains out of engagement with locking spring 308; however toggle finger 228 starts to engage first angled camming surface 310 of toggle wheel 302. Note that toggle wheel is not yet rotated so that surgical needle 212 remains clamped in lower jaw 210. In this position of upper arm 204, the ratchet mechanism is engaged as pawl 226 has completed its travel past projection 242, is no longer forced into recess 243, and has snapped proximally into engagement with the teeth of ratchet 244. Thus, the ratchet mechanism will maintain upper arm 204 and lower jaw 210 together until the completion of the stroke and the transfer of the surgical needle 212 to the opposing jaw. Moreover, when the upper arm 204 is in the position of Fig. 12, camming surface 404 of the camming projection 402 of the jaw retaining mechanism engages the projection 403 of retaining arm 401 to begin to bias retaining arm 401 proximally to hold ledge 408 on shelf 410. This positively locks the upper and lower jaws 206, 210 together to prevent separation during the remainder of the stroke. Note that camming projection 402 is retained in this position due to the engagement of pawl 226 and rack 244 on the other side of the apparatus which locks the upper arm 204 with respect to lower jaw 210.

When upper arm 204 is further squeezed towards lower arm 208, as shown in Fig. 13, release finger 230 forces locking leg 324 of locking spring 308 out of engagement with the first locking notch 316 to free toggle wheel 302 for movement. At this time, toggle leg 228 engages first angled camming surface 310 and begins to slide towards ledge 311. When the toggle mechanism is in this position, ledge 408 is retained on shelf 410 and pawl 226 is engaged in the lower teeth of rack 244, still maintaining the jaws 206, 210 in the closed position. As the arm 204 moves to the fully closed (depressed) position, toggle finger 228 engages first ledge 311 to rotate the toggle wheel 302 counterclockwise as shown in Fig. 14. As toggle wheel 302 rotates counterclockwise, abutment wheel 304 likewise rotates in the same direction to force lower blade 350 distally (see arrow D) against the force of coil spring 352 to release the surgical needle 212 from lower jaw 210. Simultaneously, upper blade 330 is allowed to return (see arrow E) to its proximal (retracted) position under the force of coil spring 325. Thus, the upper blade 330 is moved to the locking position to clamp the needle 212 in the upper jaw 206 and the lower blade 350 moves into slot 351 to release the needle 212 from the lower jaw 210 (Fig. 14B). As toggle wheel 302 is moved to its counterclockwise position, knock off pin 318 keeps release finger 230 away from locking leg 324 of locking spring 308, thus enabling the locking leg 324 to engage the second locking notch 314. Toggle wheel 302 (and abutment wheel 304) are thereby locked in this second (counterclockwise) position and thus upper blade 330 is locked in the proximal position to retain surgical needle 212. That is, since movement of the toggle wheel 302 is prevented, movement of the upper blade 330 to a release position is also prevented.

As further illustrated in Fig. 14, in this counterclockwise position of the toggle mechanism, i.e. when the toggle wheel 302 is locked in the second position so that the blades 330, 350 are locked, the pawl 226 is disengaged from rack 244 and can travel along the back side of the rack 244 to thereby allow the jaws 206, 210 to move to the open position. The position of arm retaining mechanism 400 at this time is illustrated in Fig. 14A. As shown, the camming projection 402 has moved from its position of Fig. 37A and slid past the projection 403 on retaining arm 401, thus allowing retaining arm 401 to spring back to its original distal position such that ledge 408 is out of engagement with shelf 410. This allows the upper jaw 206 to return to the open position.

Fig. 15 illustrates the position of the toggle mechanism and the ratchet mechanism when the jaws 206, 210 and arms 204, 208 have returned to the open position with the surgical needle 212 secured in the upper jaw 206. As noted, the pawl 226 of the ratchet mechanism returns to the same position as in Fig. 6 (the jaw retaining mechanism also returns to the original position of Fig. 9). However, the toggle wheel 302 is maintained in the counterclockwise position by the engagement of locking leg 324 of locking spring 308 with second locking notch 314.

If it is desired to transfer the needle 212 back to the lower jaw 210, the arms 204, 210 are closed, causing the jaw retaining mechanism and ratchet mechanism to engage as described above. Furthermore, the toggle finger 228 and release finger 230 will force the toggle wheel 302 and associated abutment wheel 304 to rotate clockwise to move the upper blade 330 distally to a release position and the lower blade 350 proximally to a clamping position to release the needle 212 from the upper jaw 206 and retain it in the lower jaw 210. The locking leg 324 of locking spring 308 will re-engage the first locking notch 316 and when the arms 204, 208 and jaws 206, 210 are open, the apparatus components will return to the position shown in Fig. 6.

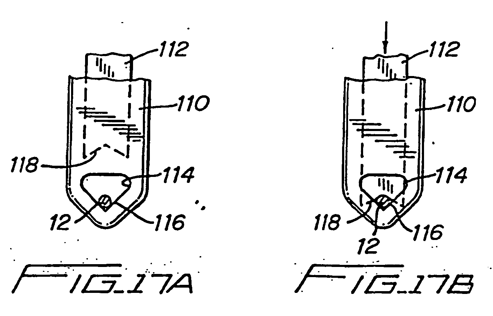

The apparatus 200 may include a quick release mechanism 500 to remove and replace or reload the surgical needle 212. In the illustrated embodiment, the needle 212 needs to be positioned in the lower jaw 210 to use the quick release mechanism, however it is also contemplated that the release mechanism can be positioned on the upper jaw which would require the needle to be in the upper jaw in order to be replaced. As shown in Figs. 2 and 16, arm 504 extends from pin 503 which is mounted in opening 294 of lower jaw 210. To replace the needle 212, lever 502 is rotated clockwise, causing arm 504 to contact portion 373 of bend 374 of the lower blade 350. This forces the blade 350 distally to release the needle 212 from the jaw 210. With the blade 350 in the distal position (see e.g. Fig. 14B), needle 212 can be removed through gap 371 between the lower blade 350 and lower jaw 210 and a fresh needle and suture can be inserted into gap 371 against raised surface 269 of lower jaw 10. Subsequently, lever 502 is released to return to its initial position of Fig. 6, allowing lower blade 350 to return to the proximal clamping position under the force of coil spring 352.

Alternate embodiments for clamping the surgical needle, which are applicable to apparatus 200 will now be described.

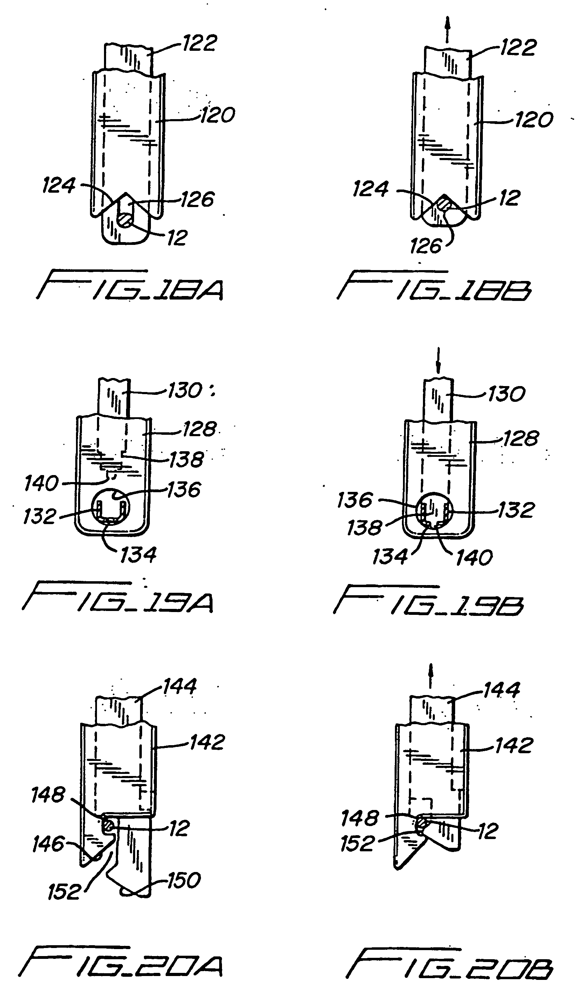

Figs. 17A and 17B illustrate an alternate embodiment of a distal end arm configuration 110 and needle engaging member 112. Arm distal end 110 includes an enlarged bore 114 for receipt of a surgical needle 12. Bore 114 aids in positioning and transferring surgical needle 12 between arms especially when pushed through tough tissue sections which may cause deflection of surgical needle 12. Bore 114 includes a V-shaped notch 116 at a distalmost end which cooperates with a V-shaped camming edge 118 on needle engaging member 112. Thus, as shown in Fig. 17B, upon distal movement of engaging member 112, V-shaped camming edge 118 cams surgical needle 12 within bore 114 against notch 116 to securely hold surgical needle 12 therein. As noted above, surgical needle 12 may be either smooth sided or notched adjacent an edge to receive at least a portion of V-shaped camming edge 118 of needle engaging member 112.

Figs. 18A and 18B illustrate an alternate arm 120 and blade or needle engaging member 122 configuration which utilizes proximal retraction, rather than distal advancement, of engaging member 122 to securely hold surgical needle 12 against arm 120. Arm 120 includes a V-shaped engagement notch 124 formed on the distal end while needle engaging member 122 contains an elongated slot 126 for receipt of surgical needle 12 therein. Referring to Fig. 18B, as blade 122 is retracted, surgical needle 12 disposed within slot 126 is cammed against and securely held within notch 124 in arm 120.

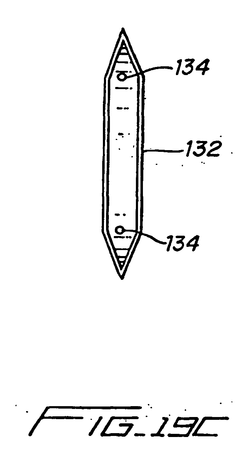

Figs. 19A and 19B illustrate yet another alternate embodiment of an arm distal end 128 and needle engaging member 130 configuration best suited for securing a U-shaped, half-circle or otherwise relatively hollow surgical needle 132 which preferably has engagement structure in the form of an engagement hole 134 formed therethrough. Surgical needle 132 (Fig. 19C) may have various cross-sectional configurations while still having suitable engagement structure in the form of hole 134. Arm 128 has a bore 136 formed therein and needle engaging member 130 has a projecting tip 138 which preferably corresponds to the interior shape of the surgical needle 132. Needle engaging member 130 is further formed with a point or finger 140 formed on tip 138 and which is specifically designed to engage the engagement structure or hole 134 in surgical needle 132. Thus, upon positioning of surgical needle 132 within recess 136, distal advancement of needle engaging member 130 causes finger 140 to engage hole 134 and securely hold surgical needle 132 within arm 128.

Referring now to Figs. 20A and 20B, an alternate arm 142 and needle engaging member 144 is shown for securely holding a round or otherwise preferably solid cross-sectional surgical needle 12 which enables easier loading. Arm 142 preferably includes an angled forward edge 146 and a groove or slot 148 proximal to angled forward edge 146. Needle engaging member 144 also includes an angled forward edge 150 and a camming member 152 formed at a distalmost portion of angled edge 150. Thus, referring to Fig. 20B, upon retraction of needle engaging member 144 camming member 152 slides past edge 146 and forces surgical needle 12 into recess slot 148, thereby securing surgical needle 12 within arm 142.

While the above described arm and needle engaging member configurations include enclosed recesses or holes through which surgical needle 12 may be perpendicularly inserted, it may often be desirable to provide open ended or easy loading structure which will allow surgical needle 12 to be inserted parallel rather than perpendicular to the arm structure. Turning first to the embodiment of Figs. 22A, 22B, arm 166 is preferably formed with a V-shaped needle guiding recess 168 having a relatively round or circular needle receiving portion 170 at the apex of the V. Thus, advancement of arm 166 distally towards surgical needle 12 will allow needle 12 to be inserted into recess 160 from the distal end of arm 166, i.e., parallel to its longitudinal axis, rather than moving surgical needle 12 perpendicularly to arm 166 to enter an enclosed recess. Needle engaging member 172 includes an angled surface 174 which, when advanced as shown in Fig. 22B, cams against surgical needle 12 to firmly hold surgical needle 12 within circular recess 170 of arm 166. It will particularly be appreciated that the easy load style of arm distal end configurations and needle engaging member configurations are particularly suited to parallel moving jaw structure which may either move perpendicular to the longitudinal axis of a surgical needle, that is, slide parallel to each other or may move parallel to the longitudinal axis of a surgical needle, i.e., that is, move perpendicular with respect to each other.

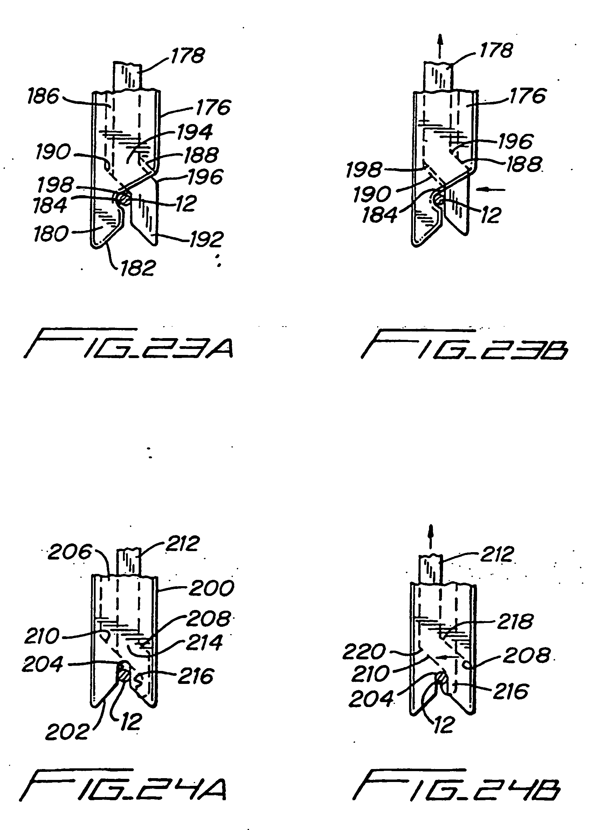

Referring now to Figs. 23A and 23B, there is disclosed a further alternate easy load style arm distal end and needle engaging member configuration. Arm 176 preferably includes a single, distally extending hook 180 having an angled needle guiding front surface 182 and small semi-circular recess 184 disposed distally of angled needle guiding front surface 182. Additionally, a channel 186 for receipt of needle engaging member 178 includes a pair of angled camming surfaces 188 and 190. Needle engaging member 178 includes a distally extending camming finger 192 having a dog leg connecting portion 194 which connects finger 192 to the remainder of needle engaging member 178. Dog leg portion 194 has camming edges 196 and 198 which cooperate with camming edges 188, 190, respectively, in arm 176. As best shown in Fig. 23B, as needle engaging member 178 is retracted, camming edge 196 abuts camming edge 188 to move finger 192 sideways forcing surgical needle 12 to be firmly held within recess 184 in arm 176. Similarly, distal advancement of needle engaging member 178 results in abutting camming edge 190 of arm 178 to engage cam edge 198 to again move finger 192 sideways away from recess 184 thereby releasing surgical needle 12 from arm 176.

Figs. 24A and 24B illustrate a further alternate embodiment of an easy load style arm and needle engaging member configuration. Arm 200 preferably includes a V-shaped needle guiding distal end 202 terminating in an elongated slot or recess 204 for receipt of surgical needle 12 therein. Preferably, arm 200 has an elongated needle engaging member channel 206 having angled edges 208 and 210. Needle engaging member 212 includes a dog leg end portion 214 similar to that described with respect to the embodiment disclosed in Figs. 23A and 23B and contains a hook or recess edge 216 at the distalmost end thereof. Thus, as shown in Fig. 24B, upon proximal retraction of needle engaging member 212, a camming edge 218 on needle engaging member 212 engages angled edge 208 on arm 200 to move needle engaging member 212 sideways thereby capturing surgical needle 12 within recess 204 by curved finger or hook 216. Similarly, distal advancement of needle engaging member 212 within channel 206 causes a camming edge 220 on needle engaging member 212 to engage angled edge 210 on arm 200 to move hook 216 away from recess 204. thereby releasing surgical needle 12 from arm 200.

Referring now to Fig. 21, an arm 154 is illustrated which is particularly suited for use with a single pointed surgical needle 12 having an associated length of suture material 14 extending from an opposite end of the point. Arm 154 is configured to manage and manipulate suture material 14 such that it does not interfere with the transfer of surgical needle 12 from an opposing arm similar to arm 16. Preferably, arm 154 includes angular inwardly sloped projecting portion 156 having a recess 158 therethrough. Recess 158 has a slot 160 along one edge thereof to allow suture material 14 to pass therethrough. Additionally, projecting portion 156 is further formed with a chamfered or channeling surface 162 which serves to guide suture material 14 through slot 160 and into recess 158. In this manner, when the surgical needle 12 is passed to arm 154, the suture will be prevented from being tangled. Additionally, the channeling surface 162, by guiding the suture material through slot 160, keeps the suture out of the way of the needle engaging member extending through slot 164. By forming the slot through arm 154, a distal end of a needle engaging member may abut surgical needle 12 to hold it within recess 158 or, alternatively, a side edge of needle engaging member may cam against an edge of surgical needle 12 to hold it within recess 158.

It will be understood that various modifications may be made to the embodiments disclosed herein. For example, as noted hereinabove, parallel movement along or perpendicular to the arm axes is contemplated as well as straight and/of double pointed surgical needles such as surgical incision members. Further, other methods of grasping a surgical needle within a single arm are contemplated. Additionally, modifications within the skill of those knowledgeable in the are may be made to the camming and reciprocating mechanisms to facilitate automatic transfer of a surgical needle from one arm to another. Therefore, the above description should not be construed as limiting, but merely as exemplifications as preferred embodiments.

## Claims

1. An apparatus for suturing tissue comprising:
a) first (204) and second (208) arms;
b) a first jaw (206) supported by the first arm and a second jaw (210) supported by the second arm, the jaws being relatively movable between an open and closed position;
c) a first needle securing member (330) movably positioned in the first jaw and a second needle securing member (350) movably positioned in the second jaw;
d) a reciprocating mechanism (302, 304) for moving the first and second needle securing members between a needle securing and a needle release position;
**characterised by**
e) a retaining mechanism (401, 410) for preventing separation of the first and second jaws during relative movement of the arms from an open position to a closed position.

2. An apparatus according to claim 1, wherein the retaining mechanism comprises an arm (401) extending from the second jaw having a hook portion (408) engagable with a shelf (410) formed in the first jaw.

3. An apparatus according to claims 1 or 2, further comprising a ratchet mechanism including a pawl (226) extending from the first arm, the pawl being engagable with a rack (244) positioned on the second jaw and further comprising a projection (242) on the second jaw for biasing the pawl into engagement with a recess (243) in the first jaw prior to engagement with the rack, thereby allowing the first arm and first jaw to initially move together towards the second jaw, the ratchet mechanism being provided for maintaining the first arm (204) and second jaw (210) together until one of the needle securing members is locked in the needle securing position.

4. An apparatus according to claim 2 further comprising a camming member (402) extending from the first arm, the camming member camming the hook portion into engagement with the shelf.

5. An apparatus for suturing tissue according to any one of the preceding claims, wherein the securing position of the first and second needle securing member is proximal of the release position.

6. An apparatus according to any one of the preceding claims, further comprising a cam actuating lever (228) operatively associated with the reciprocating mechanism (302, 304) such that the cam actuating lever cams the reciprocating mechanism between the first and second positions.

7. The apparatus according to claim 6, wherein the cam actuating lever automatically cams the reciprocating mechanism between the first and second position when the first arm is moved to a closed position relative to the second arm.

8. The apparatus according to any one of the preceding claims, wherein the reciprocating mechanism includes a toggle wheel (302) rotatably mounted on one of the first and second jaws, and wherein the cam actuating lever is mounted on one of the first and second arms, such that relative movement of the arms to the closed position drives the cam actuating lever into engagement with a camming surface on the toggle wheel to rotate the toggle wheel.

9. The apparatus according to claim 8, wherein the reciprocating mechanism includes a pivotable wheel (304) connected to the toggle wheel (302) and a proximal end of each of the first and second needle securing members abuts the pivotable wheel such that rotation of the toggle wheel simultaneously rotates the pivotable wheel to move one of the needle securing members to the securing position and the other needle securing member to the release position.

10. The apparatus according to any one of the preceding claims, further comprising a lockout member (324) operatively associated with at least one of the first and second arms and mounted for movement between a locked position blocking movement of the reciprocating mechanism and an unlocked position allowing movement of the reciprocating mechanism.

11. The apparatus according to claims 6 and 10, wherein the cam actuating lever includes a release leg (228), the release leg moving the lockout member from the locked position to the unlocked position upon relative movement of the arms to the closed position.

12. The apparatus according to any one of the preceding claims, further comprising first (325, 352)and second springs for biasing the respective first and second needle securing members proximally.

13. An apparatus according to any one of the preceding claims, wherein the reciprocating mechanism comprises a toggle wheel (302) having first and second notches (316, 314) and further comprising a locking spring (308) engagable with the first notch (316) to lock the first needle securing member in the securing position and engagable with the second notch (314) to lock the second needle securing member in the securing position.

## Patentansprüche

1. Vorrichtung zum Nähen von Gewebe mit:
a) einem ersten (204) und einem zweiten (208) Arm;
b) einer ersten Backe (206), die von dem ersten Arm gehalten ist, und einer zweiten Backe (210), die von dem zweiten Arm gehalten ist, wobei die Backen zwischen einer offenen und einer geschlossenen Position relativ bewegbar sind;
c) einem ersten Nadelanbringelement (330), das in der ersten Backe bewegbar positioniert ist, und einem zweiten Nadelanbringelement (350), das in der zweiten Backe bewegbar positioniert ist;
d) einem Bewegungsmechanismus (302, 304) zur Bewegung des ersten und des zweiten Nadelanbringelementes zwischen einer Nadelanbring- und einer Nadelfreigabeposition;
**gekennzeichnet durch**
e) einen Haltemechanismus (401, 410) zum Verhindern der Trennung der ersten und der zweiten Backe bei der Relativbewegung der Arme von einer offenen Position zu einer geschlossenen Position.

2. Vorrichtung nach Anspruch 1, bei der der Haltemechanismus einen Arm (401) aufweist, der sich von der zweiten Backe erstreckt und einen Hakenabschnitt (408) besitzt, der mit einem in der ersten Backe gebildeten Absatz (410) in Eingriff bringbar ist.

3. Vorrichtung nach Anspruch 1 oder 2, des weiteren mit einem Ratschenmechanismus, der eine Sperrklinke (226) umfasst, die sich von dem ersten Arm erstreckt, wobei die Sperrklinke mit einem auf der zweiten Backe positionierten Steg (244) in Eingriff bringbar ist, und des weiteren mit einem Vorsprung (242) auf der zweiten Backe zum Vorspannen der Sperrklinke in Eingriff mit einer Aussparung (243) in der ersten Backe vor dem Eingriff mit dem Steg, wodurch gestattet wird, dass der erste Arm und die erste Backe sich anfänglich zusammen zu der zweiten Backe hin bewegen, und wobei der Ratschenmechanismus vorgesehen ist, um den ersten Arm (204) und die zweite Backe (210) so lange zusammenzuhalten, bis eines der Nadelanbringelemente in der Nadelanbringposition arretiert ist.

4. Vorrichtung nach Anspruch 2, des weiteren mit einem Nockenelement (402), das sich von dem ersten Arm erstreckt, wobei das Nockenelement den Hakenabschnitt in Eingriff mit dem Absatz verschiebt.

5. Vorrichtung zum Nähen von Gewebe nach einem der vorhergehenden Ansprüche, bei der die Anbringposition des ersten und des zweiten Nadelanbringelementes proximal von der Freigabeposition ist.

6. Vorrichtung nach einem der vorhergehenden Ansprüche, des weiteren mit einem Nockenbetätigungshebel (228), der betriebsmäßig dem Bewegungsmechanismus (302, 304) zugeordnet ist, so dass der Nockenbetätigungshebel den Bewegungsmechanismus zwischen der ersten und der zweiten Position verschiebt.

7. Vorrichtung nach Anspruch 6, bei der der Nockenbetätigungshebel automatisch den Bewegungsmechanismus zwischen der ersten und der zweiten Position verschiebt, wenn der erste Arm zu einer geschlossenen Position relativ zu dem zweiten Arm bewegt wird.

8. Vorrichtung nach einem der vorhergehenden Ansprüche, bei der der Bewegungsmechanismus ein Kipprad (302) umfasst, das drehbar an der ersten oder der zweiten Backe befestigt ist, und bei der der Nockenbetätigungshebel an dem ersten oder dem zweiten Arm befestigt ist, so dass eine Relativbewegung der Arme zu der geschlossenen Position hin den Nockenbetätigungshebel in Eingriff mit einer Nockenoberfläche auf dem Kipprad treibt, um das Kipprad zu drehen.

9. Vorrichtung nach Anspruch 8, bei der der Bewegungsmechanismus ein drehbares Rad (304) umfasst, das mit dem Kipprad (302) verbunden ist, und ein proximales Ende eines jeden der beiden Nadelanbringelemente an das drehbare Rad angrenzt, so dass eine Drehung des Kipprades gleichzeitig das drehbare Rad dreht, um eines der Nadelanbringelement zu der Anbringposition und das andere Nadelanbringelement zu der Freigabeposition zu bewegen.

10. Vorrichtung nach einem der vorhergehenden Ansprüche, des weiteren mit einem Arretierelement (324), das betriebsmäßig zumindest einem der beiden Arme zugeordnet ist und zur Bewegung zwischen einer arretierten Position, in der eine Bewegung des Bewegungsmechanismus blockiert ist, und einer nicht arretierten Position, in der eine Bewegung des Bewegungsmechanismus möglich ist, befestigt ist.

11. Vorrichtung nach den Ansprüchen 6 und 10, bei der der Nockenbetätigungshebel ein Freigabebein (228) umfasst, wobei das Freigabebein das Arretierelement von der arretierten Position zu der nicht-arretierten Position auf eine Relativbewegung der Arme zu der geschlossenen Position hin bewegt.

12. Vorrichtung nach einem der vorhergehenden Ansprüche, des weiteren mit einer ersten (325, 352) und einer zweiten Feder zum Vorspannen des jeweiligen ersten und des zweiten Nadelanbringelementes in proximaler Richtung.

13. Vorrichtung nach einem der vorhergehenden Ansprüche, bei der der Bewegungsmechanismus eine Kipprad (302) mit einer ersten und einer zweiten Kerbe (316, 314) aufweist, und des weiteren mit einer Arretierfeder (308), die mit der ersten Kerbe (316) in Eingriff bringbar ist, um das erste Nadelanbringelement in der Anbringposition zu arretieren, und die mit der zweiten Kerbe (314) in Eingriff bringbar ist, um das zweite Nadelanbringelement in der Anbringposition zu arretieren.

## Revendications

1. Appareil de suture de tissu comprenant:
a) des premier (204) et second (208) bras;
b) une première mâchoire (206) supportée par le premier bras et une seconde mâchoire (210) supportée par le second bras, les mâchoires étant déplaçables relativement entre des positions ouverte et fermée;
c) un premier élément de fixation d'aiguille (330) positionné d'une manière mobile dans la première mâchoire et un second élément de fixation d'aiguille (350) positionné d'une manière mobile dans la seconde mâchoire;
d) un mécanisme de va-et-vient (302, 304) pour déplacer les premier et second éléments de fixation d'aiguille entre une position de fixation d'aiguille et une position de libération d'aiguille; **caractérisé par**
e) un mécanisme de retenue (401, 410) pour empêcher la séparation des première et seconde mâchoires pendant un mouvement relatif des bras d'une position ouverte à une position fermée.

2. Appareil selon la revendication 1, où le mécanisme de retenue comprend un bras (401) s'étendant depuis la seconde mâchoire, comportant une portion de crochet (408) pouvant être mise en prise avec un rebord (410) formé dans la première mâchoire.

3. Appareil selon les revendications 1 ou 2, comprenant en outre un mécanisme de verrouillage incluant un cliquet (226) s'étendant à partir du premier bras, le cliquet pouvant s'engager dans une crémaillère (244) positionnée sur la seconde mâchoire et comprenant en outre une saillie (242) sur la seconde mâchoire pour solliciter le cliquet en prise avec un évidement (243) dans la première mâchoire avant la mise en prise avec la crémaillère, en permettant ainsi au premier bras et à la première mâchoire de se déplacer initialement ensemble vers la seconde mâchoire, le mécanisme de verrouillage étant réalisé pour maintenir le premier bras (204) et la seconde mâchoire (210) ensemble jusqu'à ce que l'un des éléments de fixation d'aiguille soit verrouillé dans la position de fixation d'aiguille.

4. Appareil selon la revendication 2, comprenant en outre un élément de came (402) s'étendant depuis le premier bras, l'élément de came amenant par effet de came la portion de crochet en prise avec le rebord.

5. Appareil de suture de tissu selon l'une des revendications précédentes, où la position de fixation des premier et second éléments de fixation d'aiguille est proximale de la position de libération.

6. Appareil selon l'une des revendications précédentes, comprenant en outre un levier d'actionnement de came (228) fonctionnellement associé au mécanisme de va-et-vient (302, 304) de telle sorte que le levier d'actionnement de came amène par effet de came le mécanisme de va-et-vient entre les première et seconde positions.

7. Appareil selon la revendication 6, où le levier d'actionnement de came amène automatiquement par effet de came le mécanisme de va-et-vient entre les première et seconde positions lorsque le premier bras est amené à une position fermée relativement au second bras.

8. Appareil selon l'une des revendications précédentes, où le mécanisme de va-et-vient comprend une roue à came (302) montée d'une manière tournante sur l'une des première et seconde mâchoires, et où le levier d'actionnement de came est monté sur l'un des premier et second bras de sorte que le mouvement relatif des bras à la position fermée amène le levier d'actionnement de came en prise avec une surface de réception de came sur la roue à came pour faire tourner la roue à came.

9. Appareil selon la revendication 8, où le mécanisme de va-et-vient comprend une roue pivotante (304) reliée à la roue à came (302), et une extrémité proximale de chacun des premier et second éléments de fixation d'aiguille bute contre la roue pivotante de sorte que la rotation de la roue à came fait tourner simultanément la roue pivotante afin d'amener l'un des éléments de fixation d'aiguille à la position de fixation et l'autre élément de fixation d'aiguille à la position de libération.

10. Appareil selon l'une des revendications précédentes, comprenant en outre un élément de verrouillage (324) fonctionnellement associé à au moins l'un des premier et second bras et installé en vue d'un mouvement entre une position verrouillée bloquant le mouvement du mécanisme de va-et-vient et une position déverrouillée permettant le mouvement du mécanisme de va-et-vient.

11. Appareil selon les revendications 6 et 10, où le levier d'actionnement de came comprend une branche de libération (228), la branche de libération amenant l'élément de verrouillage de la position verrouillée à la position déverrouillée lors d'un mouvement relatif des bras à la position fermée.

12. Appareil selon l'une des revendications précédentes, comprenant en outre des premier (325, 352) et second ressorts pour solliciter les premier et second éléments de fixation d'aiguille respectifs proximalement.

13. Appareil selon l'une des revendications précédentes, où le mécanisme de va-et-vient comprend une roue à came (302) ayant des première et seconde encoches (316, 314) et comprenant en outre un ressort de verrouillage (308) pouvant être mis en prise avec la première encoche (316) pour verrouiller le premier élément de fixation d'aiguille dans la position de fixation et pouvant être mis en prise avec une seconde encoche (314) pour verrouiller le second élément de fixation d'aiguille dans la position de fixation.
